# EUROPEAN PATENT SPECIFICATION

(11) **EP 2 070 488 B1**
(45) Date of publication and mention of the grant of the patent: **08.01.2014**
(21) Application number: 08000207.4
(22) Date of filing: 20.11.2001
(51) Int. Cl.: A61B 19/02

(54) **Clear medical packaging**
Durchsichtige medizinische Produktverpackung
Emballage médical transparent

(30) Priority: 20.11.2000 US 716619; 20.11.2000 US 716687
(43) Date of publication of application: 17.06.2009
(62) Divisional of application: 01997438.5
(73) Proprietor: Becton, Dickinson and Company, Franklin Lakes, NJ 07417 (US)
(72) Inventor: Heyman, Peter W., Florham Park, NJ 07932-2653 (US); Korisch, Marina S., Wayne, NJ 07470-6414 (US); Alchas, Paul G., Franklin Lakes, NJ 07417-1815 (US)
(74) Representative: Shanks, Andrew

(56) References cited:
- EP-A- 0 266 688
- DE-A1- 2 952 733
- US-A- 4 402 407

## Description

### FIELD OF THE INVENTION

The present invention relates to medical device packages. More particularly, the present invention relates to medical device packages having a gas permeable lid and a clear plastic tub.

### BACKGROUND OF THE INVENTION

Medical devices, such as prefillable syringes and the like are frequently packaged in multi-unit packages that must be surface decontaminated prior to introduction into a controlled sterile environment where the devices are filled with medication or otherwise further manipulated prior to use, or used in a surgical procedure.

There are numerous methods used to sterilize the devices within the packages, such as gas sterilization utilizing ethylene oxide or steam sterilization. However, different methods, such as ultraviolet light sterilization, are often used for decontamination of the surfaces of the packages prior to subsequent use. Packages of this type are disclosed in US4402407, EP0266688 and DE2952733. It would be desirable to have a medical device package capable of use in a multitude of sterilization and decontamination techniques and yield a high level of kill of microorganisms.

A common type of package utilized in the field of medical packaging is the peel-open package. Such a package commonly comprises a thermoplastic film that is formed to a desired package shape and a lid material is then sealed to the plastic film to contain the packaged product.

The common thermoplastic film packages described above are often utilized to package individual medical devices, as the film does not provide sufficient protective characteristics for use in a multiunit package which may house delicate items. Therefore, there is a need for a multiunit package, such as a tub, that can withstand the sterilization and decontamination processes and provide a sufficient mechanical barrier to prevent damage to the items held within the package.

The lid material used is commonly a non-woven fiber arranged such that it has a microporous structure, such as Tyvek®. The microporous fiber arrangement allows gas to penetrate but has a sufficiently small pore size to block the transfer of microorganisms.

The microporous fiber arranged lid is, therefore, useful in sterilization techniques utilizing a gas, such as the aforementioned ethylene oxide and steam sterilization techniques.

The Tyvek® lid, however, does not allow the transmission of ultraviolet light that may be used as a package surface decontamination technique. Specifically, the heat seal between the lid and tub cannot be made flush with the edge of the tub flange duc to processing limitations and the potential for Tyvek® tearing during peel open if an unsealed lip of Tyvek® is not available for initiation of the peeling process. Therefore, there is a region outside of the heat seal, covered by Tyvek®, which is not maintained sterile with the contents of the package. This area can be inadvertently contaminated during manipulation of the package, as can the entire external surface of the package. However, unlike the remainder of the external surface, which can be directly exposed to ultraviolet light for decontamination, the area outside of the heat seal is blocked to the ultraviolet light by the opaque Tyvek® material on top and opaque tub material below.

Therefore, it would be desirable to utilize an ultraviolet-light transmissive plastic tub that can allow transmission of light to the underside of the heat seal area between the lid and tub to kill all microorganisms residing therein. Plastics which transmit light in the ultraviolet range typically transmit an even greater percentage of incident light in the visible (400-700 nanometer) range, resulting in a clear appearance.

An advantage of utilizing a clear plastic tub, as opposed to an opaque one is that it would allow for visual inspection of the contents of the package to determine if any of the items are damaged. This aspect offers quality control advantages to a manufacturer, as well as allow end users to ensure they are not receiving or using damaged items. Another advantage of utilizing a clear plastic tub would be the possible use of an automated visual quality system. For example, a package and its contents may be imaged and compared to a stored visual image to determine if the contents of the package are damaged. Such an automated system could provide a cost savings, as well as an increased quality control efficiency.

Alternatively, a Tyvek® lid having an ultraviolet-light transmitting plastic border that is heat sealed to a plastic tub would allow for light sterilization of the area between the lid and tub. Such a lid may be utilized with either a clear plastic tub, or with an opaque tub and allow for light to penetrate the area around the heat seal.

Another problem associated with Tyvek® lids that are known in the art, is the possibility of the Tyvek® lid shearing or tearing when peeled to open the package and thereby generating unacceptable particulate matter that may contaminate the contents of the package.

It is known in the art to coat a Tyvek® lid in an effort to reduce particulate generation, as well as prevent the lid from separating or delaminating when the heat seal strength between the lid and tub is too great. A disadvantage to this coating technique is that it has the tendency to reduce the pore size of the Tyvek® material; thereby preventing the necessary transmission of gas across the lid material or increasing the cycle time of a sterilization procedure.

It would be desirable to have a gas permeable uncoated Tyvek® lid that has a smooth continuous plastic border that is resistant to shearing and does not generate particulate matter when peeled away to access the contents.

A lid with a smooth continuous plastic border would also be desirable for use with an automated lid removal system, such as a hot knife or laser cutter that would cut the plastic border inside of the heat seal region, as an alternative to a peel-open technique.

### SUMMARY OF THE INVENTION

Therefore, it is an object of the present invention to provide a medical device package according to claim 1 which has an ultraviolet-transmissive and clear plastic tub to allow for visual inspection of the contents, as well as to allow for the transmission of microorganism killing light.

It is a further object of the present invention to provide a medical device package having a clear plastic tub that has sufficient strength characteristics to hold and protect from damage the contents of the package.

It is a further object of the present invention to provide a medical device package having a microporous, gas permeable lid that has a plastic border to minimize the creation of particulate matter when opening the package.

It is a further object of the present invention to provide a medical device package having a microporous, gas permeable lid with an ultraviolet-transmissive plastic border to facilitate the transmission of light during a decontamination process.

The present invention meets these objects by providing a medical device package having a tub having a four sided base from which four walls extend upwardly at approximately ninety degrees. According to the invention the walls terminate at a first flange which extends outwardly at an angle of approximately ninety degrees from the four walls. The first flange terminates at four top walls that extend upwardly at approximately ninety degrees from the flange. The four top walls terminate at a second outwardly extending flange, which extends at an angle of approximately ninety degrees from the four top walls. The tub is formed of an ultraviolet-light transmitting plastic that is capable of withstanding exposure to gas sterilization and surface decontamination processes without degradation of the tub.

The present invention also includes a lid having a gas permeable central portion and a transparent plastic film attached to the central portion along its periphery. The plastic film overlaps the central portion when attached to assure a reliable bond of the two items. The transparent plastic film has a high shear resistance to avoid producing particulate matter when an opening force is applied. The transparent plastic film also allows transmission of ultraviolet light. These and other features of the present invention can be best understood from the following specification and drawings.

### BRIEF DESCRIPTION OF THE DRAWINGS

Figure 1 is a perspective view of the medical device package of the present invention.
Figure 2 is a sectional view of the medical device package of the present invention.

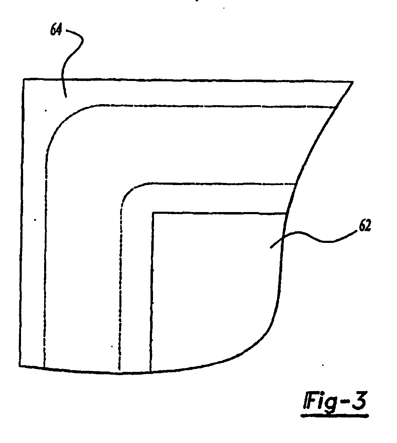

Figure 3 is a plan view of a corner of the medical device package of the present invention.
Figure 4 is perspective view of the tub of the present invention.
Figure 5 is a plan view of the tub of the present invention showing the contours that facilitate release of the tub from a mold.

### DETAILED DESCRIPTION OF THE PREFERRED EMBODIMENT

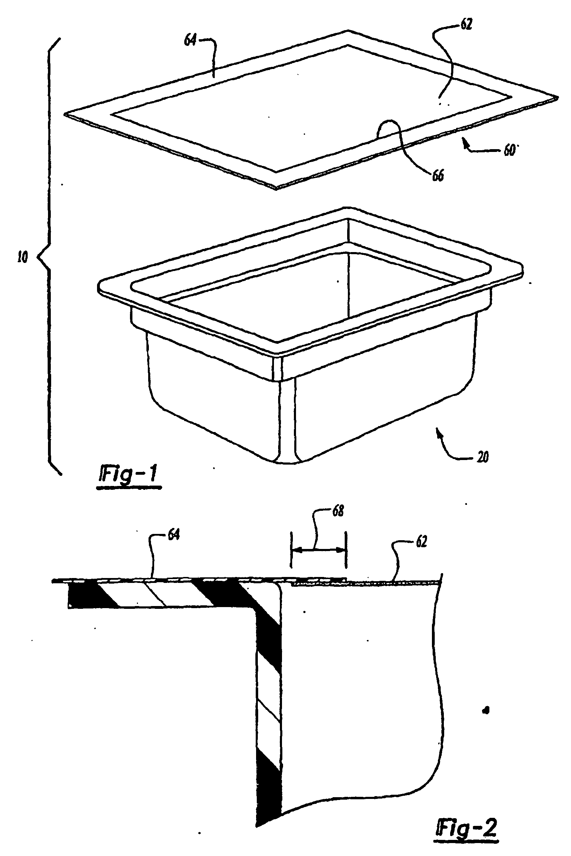

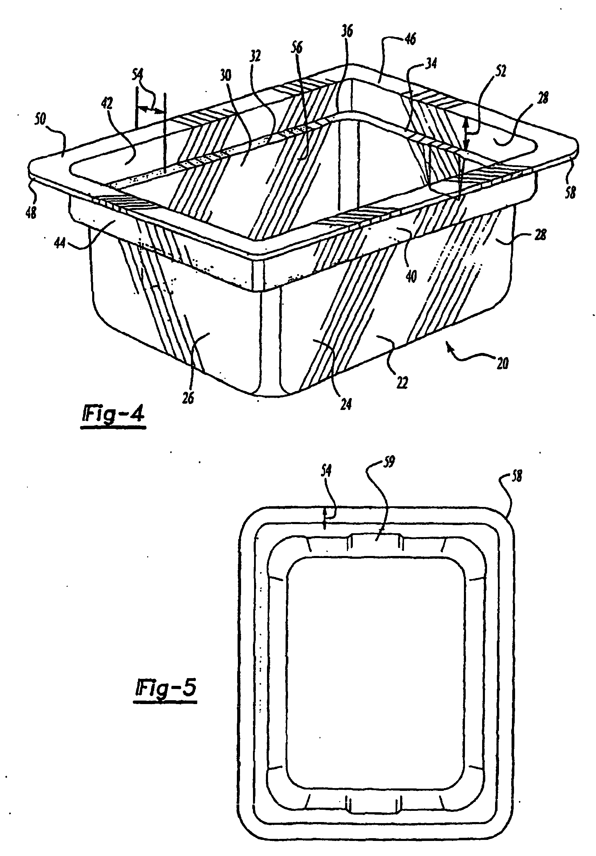

There is shown generally in Figure 1 at 10 the medical device package of the present invention. The medical device package 10 includes a tub 20 and a lid 60. With reference to Figures 1 and 4, the tub 20 has a planar base 22 from which four walls 24, 26, 28, 30 extend upward at approximately ninety degrees. The four walls 24, 26, 28, 30 terminate at an upper end 32 to form a first outwardly extending flange 34. The first flange intersects the upper end 32 of the walls at approximately ninety degrees and extends outwardly therefrom. There are four top walls 38, 40, 42, 44 extending upwardly at approximately ninety degrees from the outer edge 36 of the first outwardly extending flange 34. The four top walls 38, 40, 42, and 44 terminate at a second upper end 46 to form a second outwardly extending flange 48. The second outwardly extending flange 48 intersects the second upper end 46 at approximately ninety degrees and extends outwardly therefrom. The second outwardly extending flange has an upper surface 50 for mounting or adhering a lid 60 to the tub 20.

The gap 52 defined by the vertical distance between the first flange 34 and second flange 48 provides an area for use of an interior holding apparatus. Such an apparatus could hold the contents, for example syringes, and maintain the spatial arrangement of the contents of the package. The gap 52 also provides a depth for an automated opening tool to be utilized without contacting and possibly damaging the contents of the tub 20.

The horizontal or outward distance between the first flange 34 and second flange 48 defines a second gap 54. The contents of the tub 20 are contained inside the space 56 defined by the four walls 24, 26, 28, 30 and are inboard of the first flange 34. The gap 54, similar to the gap 52 defines a buffer zone when utilizing an automated opening tool such as a hot knife, or laser cutter to prevent damage of the contents of the tub 20.

The tub 20 is preferably formed of a plastic resin that allows the transmission of both visible and ultraviolet light. Examples of suitable materials for visible light transmission only, include high melt flow polycarbonate and high melt flow copolyester, which also exhibit good impact strength and overall durability. A particularly preferred resin of the present invention is methylpentene copolymer which transmits both visible and ultraviolet light and provides good mechanical protection of the contents of the tub. Specifically, the methylpentene copolymer demonstrates good transmissive properties at the microorganism killing ultraviolet wavelengths at 254 nanometers.

The tub 20 is preferably formed utilizing an injection molding process or a reaction injection molding process. In an injection molding process, the resin is injected into a mold under a specified temperature and pressure and allowed to cure to form a solid article.

The tub 20 of the present invention, preferably, has radiused corners 58 both on its exterior and interior to facilitate removal of the tub 20 from the mold during manufacture. The radiused corners 58 also prevent the formation of sharp edges that may cause damage to the contents of the tub 20, as well as, pose a threat of possible injury to a person handling the tub 20. The tub 20 may also include contoured portions 59, as shown in Figures 4 and 5, to facilitate removal of the tub 20 from the mold during manufacture.

The lid 60 of the present invention comprises a gas permeable microfiber central portion 62 and a transparent plastic film 64 attached to the central portion 62 along its periphery 66.

The central portion 62 preferably comprises Tyvek®, a polyolefin microfiber material produced by E.I. Dupont de Nemours and Company of Wilmington, Delaware. Even more preferably, the central portion 62 comprises uncoated Tyvek® 1073B.

The transparent plastic film 64 is attached along the periphery 66 of the central portion using adhesives, glues or other bonding agents or may be heat welded. The transparent plastic film 64 should overlap 68 the central portion at least five millimeters to assure an adequate bonding between the two items.

The width of the transparent film 64 is variable, dependent upon the application to which it is to be associated. For example, if the lid 60 is to be removed manually utilizing a peel-away procedure, a narrow width in the range of 10-20 millimeters may be utilized The 10-20 millimeter width would allow sufficient light to enter to sterilize the area just outside of the heat seal, but not be excessively wide from an economic perspective. If the lid 60 is to be utilized in an alternate application such as an automated opening operation, a wider width may be utilized to prevent possible shearing of the central portion 62, thereby producing particulate matter. A suitable width for the transparent film 64 is in the range of 20 to 30 millimeters for an automated procedure.

The film 64 should allow transmission of light in the ultraviolet spectrum for surface decontamination procedures. The film 64 should also have a sufficient shear strength to resist accidental tearing, but be capable of easy opening utilizing an automated system.

The present invention has been described in accordance with the relevant legal standards, thus the foregoing description is exemplary rather than limiting in nature. Variations and modifications to the disclosed embodiment may become apparent to those skilled in the art. Accordingly, the scope of legal protection afforded this invention can only be determined by studying the following claims.

## Claims

1. A medical device package (10) comprising:
a tub (20) having a four-side base (22); and
four walls (24, 26, 28, 30) intersecting the base at approximately ninety degrees and extending therefromand terminating at a first outwardly extending flange (34) which extends from the four walls (24, 26, 28, 30) at approximately ninety degrees;
said tub (20) being formed of clear plastic that is capable of withstanding exposure to steam and ethylene oxide sterilization environments without degradation of the tub;
**characterized in that** the first outwardly extending flange (34) terminates at four top walls (38, 40, 42, 44) that extend from the first outwardly extending flange (34) at approximately ninety degrees and terminate at a second outwardly extending flange (48) that extends from the four top walls (38, 40, 42, 44) at approximately ninety degrees.

2. The medical device package (10) of claim 1, wherein:
the four walls (24, 26, 28, 30) extend upwardly from the base (22); and
said plastic permits passage of ultraviolet light through said walls of said tub (20) for sterilization of a medical device in said tub (20); and
further comprising a lid (60) including a gas permeable central portion (62) framed in a border (66) of plastic film for permitting passage of disinfectant gas through said lid (60), said plastic film being in sealing engagement with said second flange (48).

3. The medical device package (10) of claim 2, wherein the second outwardly extending flange (48) extends outward a greater distance than the first outwardly extending flange (34).

4. The medical device package (10) of claim 2, wherein a vertical distance from the first outwardly extending flange (34) and the second outwardly extending flange (48) defines a gap (52) for use of an interior holding apparatus.

5. The medical device package (10) of claim 2, wherein a horizontal distance from the first outwardly extending flange (34) and the second outwardly extending flange (48) defining a second gap (54) to provide a buffer zone when utilizing an automated opening procedure.

6. The medical device package (10) of claim 2, wherein the tub (20) comprises at least one of: high melt flow polycarbonate; high melt flow copolyester; methylpentene copolymer; and a thermoplastic.

7. The medical device package (10) of claim 2, wherein the tub (20) further includes at least one of: radiused corners (58) to facilitate removal of the tub (20) from a mold, and prevent the formation of sharp edges; and contoured portions (59) to facilitate removal of the tub (20) from a mold.

8. The medical device package (10) of claim 2, wherein the tub (20) comprises a plastic which permits passage of light ranging from 400 to 700 nanometers.

9. The medical device package (10) of claim 1, further comprising:
a lid (60) having a gas permeable central portion (62); and
a transparent plastic film (64) attached to the central portion (62) along a periphery (66) of the central portion (62) and said plastic film (64) overlapping (68) said central portion (62), said transparent plastic film (64) having a high shear resistance to avoid producing particulate matter when a force is applied and said transparent film (64) allowing transmission of light in the ultraviolet wavelengths to decontaminate or sterilize an area beneath the transparent film (64).

10. The medical device package (10) of claim 9, wherein the central portion (62) comprises Tyvek®.

11. The medical device package (10) of claim 9, wherein the central portion (62) and transparent plastic film (64) overlap (68) at least five millimetres.

12. The medical device package (10) of claim 9, wherein the transparent plastic film (64) has a width of from 10 to 20 millimetres, or from 20 to 30 millimetres.

13. The medical device package (10) of claim 1, wherein:
the walls(24, 26, 28, 30) intersecting the base (22) are side walls; and
further comprising a lid (60) mounted to said second flange (48), said lid (60) having a gas permeable central portion (62) and transparent plastic film (64) attached to the central portion (62); and
said transparent plastic film (64) attached to the central portion (62) along a periphery (66) of the central portion (62) and said plastic film (64) overlapping (68) said central portion (62) said transparent plastic film (64) having a high shear resistance to avoid producing particulate matter when a force is applied and said transparent film (64) allowing transmission of light in the ultraviolet wavelengths to decontaminate or sterilize an area beneath the transparent film (64).

14. The medical device package (10) of claim 13, wherein the second outwardly extending flange (48) extends outward a greater distance than the first outwardly extending flange (34).

15. The medical device package (10) of claim 13, wherein a vertical distance from the first outwardly extending flange (34) and the second outwardly extending flange (48) defines a gap (52) for use of an interior holding apparatus.

16. The medical device package (10) of claim 13, wherein a horizontal distance from the first outwardly extending flange (34) and the second outwardly extending flange (48) defining a second gap (54) to provide a buffer zone when utilizing an automated opening procedure.

17. The medical device package (10) of claim 13, wherein the tub (20) comprises one of: high melt flow polycarbonate; high melt flow copolyester; methylpentene copolymer; and a thermoplastic.

18. The medical device package (10) of claim 13, wherein the tub (20) further includes at least one of:
radiused corners (58) to facilitate removal of the tub (20) from a mold, and prevent the formation of sharp edges; and
contoured portions (59) to facilitate removal of the tub (20) from a mold.

19. The medical device package (10) of claim 13, wherein the tub (20) is integrally formed.

20. The medical device package (10) of claim 13, wherein the tub (20) comprises a plastic which permits passage of light ranging from 400 to 700 nanometers.

## Patentansprüche

1. Medizinische Geräteverpackung (10), die Folgendes umfasst:
eine Wanne (20), die eine Vierseitenbasis (22) hat, und
vier Wände (24, 26, 28, 30), welche die Basis mit ungefähr neunzig Grad überschneiden und sich von derselben aus erstrecken und an einem ersten sich nach außen erstreckenden Flansch (34) enden, der sich von den vier Wänden (24, 26, 28, 30) aus mit ungefähr neunzig Grad erstreckt;
wobei die Wanne (20) aus einem klaren Kunststoff geformt ist, der in der Lage ist, ohne Zersetzung der Wanne einer Aussetzung gegenüber Dampf- und Ethylenoxid-Sterilisationsumgebungen standzuhalten;
**dadurch gekennzeichnet, dass** der erste sich nach außen erstreckende Flansch (34) an vier oberen Wänden (38, 40, 42, 44) endet, die sich von dem ersten sich nach außen erstreckenden Flansch (34) aus mit ungefähr neunzig Grad erstrecken und an einem zweiten sich nach außen erstreckenden Flansch (48) enden, der sich von den vier oberen Wänden (38, 40, 42, 44) aus mit ungefähr neunzig Grad erstreckt.

2. Medizinische Geräteverpackung (10) nach Anspruch 1, wobei:
sich die vier Wände (24, 26, 28, 30) von der Basis (22) aus nach oben erstrecken und
der Kunststoff einen Durchgang von ultraviolettem Licht durch die Wände der Wanne (20) zum Sterilisieren eines medizinischen Gerätes in der Wanne (20) ermöglicht und
die ferner einen Deckel (60) umfasst, der einen gasdurchlässigen Mittelabschnitt (62) einschließt, der eingerahmt ist in einem Rand (66) aus Kunststoff-Folie, um einen Durchgang eines desinfizierenden Gases durch den Deckel (60) hindurch zu ermöglichen, wobei sich die Kunststoff-Folie in abdichtendem Eingriff mit dem zweiten Flansch (48) befindet.

3. Medizinische Geräteverpackung (10) nach Anspruch 2, wobei sich der zweite sich nach außen erstreckende Flansch (48) um eine größere Strecke nach außen erstreckt als der erste sich nach außen erstreckende Flansch (34).

4. Medizinische Geräteverpackung (10) nach Anspruch 2, wobei ein vertikaler Abstand von dem ersten sich nach außen erstreckenden Flansch (34) zu dem zweiten sich nach außen erstreckenden Flansch (48) einen Spalt (52) zur Verwendung einer inneren Haltevorrichtung definiert.

5. Medizinische Geräteverpackung (10) nach Anspruch 2, wobei ein horizontaler Abstand von dem ersten sich nach außen erstreckenden Flansch (34) zu dem zweiten sich nach außen erstreckenden Flansch (48) einen zweiten Spalt (54) definiert, um eine Pufferzone bereitzustellen, wenn ein automatisiertes Öffnungsverfahren benutzt wird.

6. Medizinische Geräteverpackung (10) nach Anspruch 2, wobei die Wanne (20) wenigstens eines der Folgenden umfasst: Polycarbonat mit hohem Schmelzindex, Copolyester mit hohem Schmelzindex, Methylpenten-Copolymer und einen thermoplastischen Kunststoff.

7. Medizinische Geräteverpackung (10) nach Anspruch 2, wobei die Wanne (20) ferner wenigstens eines der Folgenden einschließt: gerundete Ecken (58), um ein Entfernen der Wanne (20) aus einer Form zu erleichtern und die Bildung von scharfen Kanten zu verhindern, und konturierte Abschnitte (59), um ein Entfernen der Wanne (20) aus einer Form zu erleichtern.

8. Medizinische Geräteverpackung (10) nach Anspruch 2, wobei die Wanne (20) einen Kunststoff umfasst, der einen Durchgang von Licht ermöglicht, das von 400 bis 700 Nanometer reicht.

9. Medizinische Geräteverpackung (10) nach Anspruch 1, die ferner Folgendes umfasst:
einen Deckel (60), der einen gasdurchlässigen Mittelabschnitt (62) hat, und
eine an dem Mittelabschnitt (62) längs eines Umfangs (66) des Mittelabschnitts (62) befestigte durchscheinende Kunststoff-Folie (64), und wobei die Kunststoff-Folie (64) den Mittelabschnitt (62) überlappt (68), wobei die durchscheinende Kunststoff-Folie (64) eine hohe Scherfestigkeit hat, um ein Erzeugen von Feststoffen zu vermeiden, wenn eine Kraft ausgeübt wird, und wobei die durchscheinende Folie (64) ein Durchlassen von Licht in den ultravioletten Wellenlängen ermöglicht, um einen Bereich unterhalb der durchscheinenden Folie (64) zu dekontaminieren oder zu sterilisieren.

10. Medizinische Geräteverpackung (10) nach Anspruch 9, wobei der Mittelabschnitt (62) Tyvek^{®} umfasst.

11. Medizinische Geräteverpackung (10) nach Anspruch 9, wobei der Mittelabschnitt (62) und die durchscheinende Kunststoff-Folie (64) einander wenigstens fünf Millimeter überlappen (68).

12. Medizinische Geräteverpackung (10) nach Anspruch 9, wobei die durchscheinende Kunststoff-Folie (64) eine Breite von 10 bis 20 Millimetern oder von 20 bis 30 Millimetern hat.

13. Medizinische Geräteverpackung (10) nach Anspruch 1, wobei:
die Wände (24, 26, 28, 30), welche die Basis (22) überschneiden, Seitenwände sind, und
die ferner einen an dem zweiten Flansch (48) angebrachten Deckel (60) umfasst, wobei der Deckel (60) einen gasdurchlässigen Mittelabschnitt (62) und eine an dem Mittelabschnitt (62) befestigte durchscheinende Kunststoff-Folie (64) hat, und
wobei die Kunststoff-Folie (64) längs eines Umfangs (66) des Mittelabschnitts (62) an dem Mittelabschnitt (62) befestigt ist und die Kunststoff-Folie (64) den Mittelabschnitt (62) überlappt (68), wobei die durchscheinende Kunststoff-Folie (64) eine hohe Scherfestigkeit hat, um ein Erzeugen von Feststoffen zu vermeiden, wenn eine Kraft ausgeübt wird, und wobei die durchscheinende Folie (64) ein Durchlassen von Licht in den ultravioletten Wellenlängen ermöglicht, um einen Bereich unterhalb der durchscheinenden Folie (64) zu dekontaminieren oder zu sterilisieren.

14. Medizinische Geräteverpackung (10) nach Anspruch 13, wobei sich der zweite sich nach außen erstreckende Flansch (48) um eine größere Strecke nach außen erstreckt als der erste sich nach außen erstreckende Flansch (34).

15. Medizinische Geräteverpackung (10) nach Anspruch 13, wobei ein vertikaler Abstand von dem ersten sich nach außen erstreckenden Flansch (34) zu dem zweiten sich nach außen erstreckenden Flansch (48) einen Spalt (52) zur Verwendung einer inneren Haltevorrichtung definiert.

16. Medizinische Geräteverpackung (10) nach Anspruch 13, wobei ein horizontaler Abstand von dem ersten sich nach außen erstreckenden Flansch (34) zu dem zweiten sich nach außen erstreckenden Flansch (48) einen zweiten Spalt (54) definiert, um eine Pufferzone bereitzustellen, wenn ein automatisiertes Öffnungsverfahren benutzt wird.

17. Medizinische Geräteverpackung (10) nach Anspruch 13, wobei die Wanne (20) eines der Folgenden umfasst: Polycarbonat mit hohem Schmelzindex, Copolyester mit hohem Schmelzindex, Methylpenten-Copolymer und einen thermoplastischen Kunststoff.

18. Medizinische Geräteverpackung (10) nach Anspruch 13, wobei die Wanne (20) ferner wenigstens eines der Folgenden einschließt:
gerundete Ecken (58), um ein Entfernen der Wanne (20) aus einer Form zu erleichtern und die Bildung von scharfen Kanten zu verhindern, und
konturierte Abschnitte (59), um ein Entfernen der Wanne (20) aus einer Form zu erleichtern.

19. Medizinische Geräteverpackung (10) nach Anspruch 13, wobei die Wanne (20) integral geformt ist.

20. Medizinische Geräteverpackung (10) nach Anspruch 13, wobei die Wanne (20) einen Kunststoff umfasst, der einen Durchgang von Licht ermöglicht, das von 400 bis 700 Nanometer reicht.

## Revendications

1. Emballage pour dispositif médical (10), comprenant :
un bac (20) comportant une base à quatre côtés (22) ; et
quatre parois (24, 26, 28, 30), coupant la base à environ quatre-vingt-dix degrés et s'étendant à partir de celle-ci et se terminant au niveau d'une première bride s'étendant vers l'extérieur (34), s'étendant à partir des quatre parois (24,26, 28, 30) à environ quatre-vingt-dix degrés ;
ledit bac (20) étant formé de plastique clair capable de résister à une exposition à des environnements de stérilisation à la vapeur et à l'oxyde d'éthylène sans provoquer de dégradation du bac ;
**caractérisé en ce que** la première bride s'étendant vers l'extérieur (34) se termine au niveau de quatre parois supérieures (38, 40, 42, 44), s'étendant à partir de la première bride s'étendant vers l'extérieur (34) à environ quatre-vingt-dix degrés et se termine au niveau d'une deuxième bride s'étendant vers l'extérieur (48), s'étendant à partir des quatre parois supérieures (38, 40, 42, 44) à environ quatre-vingt-dix degrés.

2. Emballage pour dispositif médical (10) selon la revendication 1, dans lequel :
les quatre parois (24, 26, 28, 30) s'étendent vers le haut à partir de la base (22) ; et
ledit plastique permet le passage de la lumière ultraviolette à travers lesdites parois dudit bac (20) en vue de la stérilisation d'un dispositif médical dans ledit bac (20) ; et
comprenant en outre un couvercle (60), englobant une partie centrale perméable au gaz (62), encadrée dans une bordure (66) d'un film plastique, pour permettre le passage de gaz désinfectant à travers ledit couvercle (60), ledit film plastique étant engagé de manière étanche dans ladite deuxième bride (48).

3. Emballage pour dispositif médical (10) selon la revendication 2, dans lequel la deuxième bride s'étendant vers l'extérieur (48) s'étend vers l'extérieur sur une distance supérieure à celle de la première bride s'étendant vers l'extérieur (34).

4. Emballage pour dispositif médical (10) selon la revendication 2, dans lequel une distance verticale entre la première bride s'étendant vers l'extérieur (34) et la deuxième bride s'étendant vers l'extérieur (48) définit un espace (52) en vue de l'utilisation d'un dispositif de retenue interne.

5. Emballage pour dispositif médical (10) selon la revendication 2, dans lequel une distance horizontale entre la première bride s'étendant vers l'extérieur (34) et la deuxième bride s'étendant vers l'extérieur (48) définit un deuxième espace (54) pour établir une zone tampon lors de l'application d'une procédure d'ouverture automatique.

6. Emballage pour dispositif médical (10) selon la revendication 2, dans lequel le bac (20) comprend au moins une des substances ci-dessous : un polycarbonate à indice de fluidité élevé ; un copolyester à indice de fluidité élevé; un copolymère de méthylpentène ; et un thermoplastique.

7. Emballage pour dispositif médical (10) selon la revendication 2, dans lequel le bac (20) englobe en outre au moins une des structures ci-dessous : des coins arrondis (58) pour faciliter le retrait du bac (20) d'un moule, et empêcher la formation d'arêtes aiguës ; et des parties profilées (59) pour faciliter le retrait du bac (20) d'un moule.

8. Emballage pour dispositif médical (10) selon la revendication 2, dans lequel le bac (20) comprend un plastique permettant le passage de la lumière d'une longueur d'onde comprise entre 400 et 700 nanomètres.

9. Emballage pour dispositif médical (10) selon la revendication 1, comprenant en outre :
un couvercle (60), comportant une partie centrale perméable au gaz (62) ; et
un film plastique transparent (64), fixé sur la partie centrale (62), le long d'une périphérie (66) de la partie centrale (62), ledit film plastique (64) chevauchant (68) ladite partie centrale (62), ledit film plastique transparent (64) présentant une résistance au cisaillement élevée pour empêcher la production de matières particulaires lors de l'application d'une force, et ledit film transparent (64) permettant la transmission de la lumière dans les longueurs d'onde de l'ultraviolet pour décontaminer ou stériliser une zone au-dessous du film transparent (64).

10. Emballage pour dispositif médical (10) selon la revendication 9, dans lequel la partie centrale (62) comprend du Tyvek®.

11. Emballage pour dispositif médical (10) selon la revendication 9, dans lequel la partie centrale (62) et le film plastique transparent (64) se chevauchent (68) sur au moins cinq millimètres.

12. Emballage pour dispositif médical (10) selon la revendication 9, dans lequel le film plastique transparent (64) a une largeur comprise entre 10 et 20 millimètres, ou entre 20 et 30 millimètres.

13. Emballage pour dispositif médical (10) selon la revendication 1, dans lequel :
les parois (24, 26, 28, 30) coupant la base (22) sont des parois latérales ; et
comprenant en outre un couvercle (60) monté sur ladite deuxième bride (48), ledit couvercle (60) comportant une partie centrale perméable au gaz (62) et un film plastique transparent (64) fixé sur la partie centrale (62) ; et
ledit film plastique transparent (64) fixé sur la partie centrale (62) le long de la périphérie (66) de la partie centrale, ledit film plastique transparent (64) chevauchant (68) ladite partie centrale (62), ledit film pastique transparent (64) présentant une résistance au cisaillement élevée pour empêcher la production de matières particulaires lors de l'application d'une force, ledit film transparent (64) permettant la transmission de la lumière dans les longueurs d'onde de l'ultraviolet pour décontaminer ou stériliser une zone au-dessous du film transparent (64).

14. Emballage pur dispositif médical (10) selon la revendication 13, dans lequel la deuxième bride s'étendant vers l'extérieur (48) s'étend vers l'extérieur sur une distance supérieure à celle de la première bride s'étendant vers l'extérieur (34).

15. Emballage pour dispositif médical (10) selon la revendication 13, dans lequel une distance verticale entre la première bride s'étendant vers l'extérieur (34) et la deuxième bride s'étendant vers l'extérieur (48) définit un espace (52) en vue de l'utilisation d'un dispositif de retenue interne.

16. Emballage pour dispositif médical (10) selon la revendication 13, dans lequel une distance horizontale entre la première bride s'étendant vers l'extérieur (34) et la deuxième bride s'étendant vers l'extérieur (48) définit un deuxième espace (54) pour établir une zone tampon lors de l'application d'une procédure d'ouverture automatique.

17. Emballage pour dispositif médical (10) selon la revendication 13, dans lequel le bac (20) comprend une des substances ci-dessous : un polycarbonate à indice de fluidité élevé ; un copolyester à indice de fluidité élevé ; un copolymère de méthylpentène ; et un thermoplastique.

18. Emballage pour dispositif médical (10) selon la revendication 13, dans lequel le bac (20) comprend en outre au moins une des structures ci-dessous :
des coins arrondis (58) pour faciliter le retrait du bac (20) d'un moule, et empêcher la formation d'arêtes aiguës ; et
des parties profilées (59) pour faciliter le retrait du bac (20) d'un moule.

19. Emballage pour dispositif médical (10) selon la revendication 13, dans lequel le bac (20) est formé d'une seule pièce.

20. Emballage pour dispositif médical (10) selon la revendication 13 ; dans lequel le bac (20) comprend un plastique permettant le passage de la lumière d'une longueur d'onde comprise entre 400 et 700 nanomètres.
